# EUROPEAN PATENT APPLICATION

(11) **EP 1 671 963 A1**
(43) Date of publication of application: **21.06.2006**
(21) Application number: 04787902.8
(22) Date of filing: 17.09.2004
(51) Int. Cl.: C07D 311/62, A61K 31/353, A61P 3/02, A61P 43/00, A23L 1/30

(54) **ENDURANCE IMPROVING AGENT**

(30) Priority: 18.09.2003 JP 2003326140
(71) Applicant: KAO CORPORATION, Tokyo 103-8210 (JP)
(72) Inventor: SHIMOTOYODOME, Akira, Kao Corporation Research Lab, Haga-Gun, Tochigi 3213497 (JP); HARAMIZU, Satoshi, Kao Corporation Research Labs, Haga-Gun, Tochigi 3213497 (JP); HARADA, Ushio, Kao Corporation Research Labs, Haga-Gun, Tochigi 3213497 (JP); MURASE, Takatoshi, Kao Corproation Research Labs, Haga-Gun, Tochigi 3213497 (JP); TOKIMITSU, Ichirou, Kao Corproation Research Labs, Haga-Gun, Tochigi 3213497 (JP)
(74) Representative: HOFFMANN EITLE
(86) International application number: PCT/JP2004/013652
(87) International publication number: WO 2005/028464

(57) **Abstract**

The present invention is useful as endurance improving agents or antifatigue agents for physical activities in broad sense, such as exercises requiring endurance, labor requiring repeated muscle exercise, and the like.

The present invention relates to endurance improving agents, antifatigue agents and AMPK activators, all of which contain catechins as an active ingredient.

## Description

### Field of the Invention

This invention relates to endurance improving agents and antifatigue agents for physical activities in broad sense, including exercises and labor.

### Background of the Invention

Improving endurance and suppressing fatigue in association with exercise or labor are strongly required for physical activities in broad sense, such as exercises requiring endurance and labor requiring repeated muscle exercise.

From such a viewpoint, a variety of probes have been conducted with the aim of obtaining ingredients effective for improving endurance and suppressing fatigue. For example, ganoderma lucidum components (Patent Document 1), *Crataegus cuneata* Sieb. et Zucc. fruit extract (Patent Document 2) and the like have been reported as ingredients capable of improving endurance. As antifatigue agents, biotin (Patent Document 3), certain amino acid compositions (Patent Document 4), 2-ketogluraric acid (Patent Document 5) and on the like have been reported.

In the meantime, catechins contained in green tea, grapes, cacao beans and the like have been reported to have physiological benefits, such as cholesterol-level-increase-inhibiting effect (Patent Document 6), α-amylase-activity-inhibiting effect (Patent Document 7), blood-sugar-level-increase-inhibiting effect (Patent Document 8), arteriosclerosis-preventing effect (Patent Document 9), antioxidative effect (Patent Document 10), antimicrobial effect (Patent Document 11), blood-pressure-increase-suppressing and enzyme-activity-inhibiting effects (Patent Document 12), antiulcer effect (Patent Document 13), and mutation-inhibiting effect. However, nothing is yet known as to what effects catechins might bring in endurance and fatigue upon exercises.
Patent Document 1: JP-A-05-123135
Patent Document 2: JP-A-08-047381
Patent Document 3: JP-A-06-305963
Patent Document 4: JP-A-07-025838
Patent Document 5: JP-A-10-175855
Patent Document 6: JP-A-60-156614
Patent Document 7: JP-A-03-133928
Patent Document 8: JP-A-04-253918
Patent Document 9: JP-A-04-352726
Patent Document 10: JP-B-01-044234
Patent Document 11: JP-A-02-276562
Patent Document 12: JP-A-03-133928
Patent Document 13: JP-A-63-277628

This invention provides an endurance improving agent and an antifatigue agent, both of which contain catechins as active ingredients.

This invention also provides use of catechins for the production of an endurance improving agent and an antifatigue agent.

This invention also provides a method for improving endurance and a method for ameliorating fatigue, both of which include administering an effective dose of catechins.
This invention further provides anAMPK activator containing catechins as active ingredients.
This invention still further provides use of catechins for the production of an AMPK activator.
This invention yet further provides a method for activating AMPK by administering an effective dose of catechins.

### Modes for Carrying out the Invention

The present inventors have conducted an investigation about physiological effects of catechins, and as a result, have unexpectedly found that catechins have excellent endurance improving effect and antifatigue effect, and also AMPK (APM-activated protein kinase)-activating effect.

According to the present invention, there are provided drug and foods, which have endurance improving effect, fatigue-suppressing effect andAMPK-activating effect for physical activities in broad sense, such as exercises requiring endurance, labor requiring repeated muscle exercise and the like, and which also have effects as substitutes for leptin and adiponectin.

In general, the term "catechins" is a generic term, which encompasses catechin, gallocatechin, catechingallate, gallocatechingallate, epicatechin, epigallocatechin, epicatechingallate and epigallocatechingallate. In the present invention, catechins may contain one or more of these compounds.

Catechins, ingredients for use in the present invention, can be extracted from tea leaves prepared from crude tea leaves of the Genus Camellia, such as C. sinensis, C. assamica or the Yabukita variety, or a hybrid thereof, with water or hot water, and in some instances, the extraction can be conducted with an extraction aid added to water or hot water. Such prepared tea leaves include (1) green teas such as *sencha* (middle-grade green tea), *bancha* (coarse green tea), *gyokuro* (shaded green tea), *tencha* (powdered tea) and *kamairicha* (roasted tea); (2) semi-fermented teas such as *tekkannon* (*Teguajin*), *irotane, ougonkei* (*huang jin gui*) and *buigancha* (*Wuyiyancha*), all of which are collectively called "oolong tea"; and (3) fermented teas called "black tea", such as Darjeeling, Ceylon Uva and Chinese Keemun. As an extraction method of tea, the extraction can be effected by a conventional method such as stirring extraction. Upon extraction, an organic acid or an organic acid salt such as sodium ascorbate may be added to water. It is also possible to make combined use of boiling deaeration or an extraction method which is conducted while bubbling an inert gas such as nitrogen gas to eliminate dissolved oxygen, that is, under a so-called non-oxidizing atmosphere. Instead of directly extracting from tea leaves, it is also possible to add a concentrate or purified product of a tea extract.

The term "the concentrate of a tea extract" as used herein means one obtained by concentrating an extract of tea leaves in hot water or a water-soluble organic solvent, while the term "the purified product of a tea extract" as used herein means one obtained by conducting purification with a solvent and a column. Examples thereof include those prepared by the processes exemplified in detail in JP-A-59-219384, JP-A-04-020589, JP-A-05-260907, JP-A-05-306279 and so on. Commercially-available products include "POLYPHENON" (product of Tokyo Food Techno Co., Ltd.), "TEAFURAN" (product of ITO EN, LTD.), "SUNPHENON" (product of Taiyo Kagaku Co., Ltd.), "SUN OOLONG" (product of Suntory Limited), etc. As catechins, on the other hand, it is possible to use products obtained from other raw material sources, for example, grapes and products obtained by processing grapes such as wine, juice or the like, cacao beans and those obtained by processing cacao beans as a raw material, and even chemically synthesized products. As the forms of a concentrate of a tea extract and a purified product of a tea extract, various forms can be mentioned such as solids, aqueous solutions and slurries. As a liquid for dissolving or diluting the concentrate of the tea extract or the purifiedproduct of the tea extract, water, carbonated water, a conventional tea extract or the like can be mentioned.

As catechins, a concentrate of a tea extract or a purified product of a tea extract is generally used. In particular, the use of a concentrate of a green tea extract or a purified product of a green tea extract is preferred.

As demonstrated in Examples to be described subsequently herein, catechins have endurance improving effect, for example, such as capability of extending the maximal running time, thereby materializing advantageous effects such as the prevention, amelioration or the like of muscle fatigue or body fatigue.

Catechins also have excellent AMPK-activating effect, as demonstrated in the Examples to be described subsequently herein.

AMPK increases its activity under such a condition as in an exercise, where the intracellular ATP level tends to decrease, and as a result, serves as a "metabolic sensor" capable of promoting metabolism and then stimulates the ATP synthesis.

AMPK is known to affect the fatty-acid oxidation in mitochondria through activity control of acetyl CoA carboxylase (ACC). Carnitine parmitoyl transferase (CPT-1) which uptakes long-chain fatty acids into mitochondria is a rate-limiting enzyme for fatty-acid oxidation, and is strongly inhibited by malonyl CoA which is produced by ACC. It is, therefore, considered that AMPK increases Ser79 phosphorylation in ACC to inhibit the activity of ACC and to reduce the amount of malonyl CoA, and as a result, the activity of CPT-1 is enhanced to promote fatty-acid oxidation. As appreciated from the foregoing, AMPK is considered not only to vitalize its activity under energy deficiency in cells, but also to play an important role in the energy metabolism and nutrient metabolism in the living body.

By recent researches, it has been found that AMPK is also activated by leptin (Nature, **415**, 339-343, 2002) or an adipocyte-derived hormone such as adiponectin (Nature, **423**, 762-769, 2003). Medically, these proteins are administered in attempts to treat various diseases.
As the activation of AMPK enhances the production of energy, the ingestion of catechins according to the present invention in a situation, where energy is required as in an exercise, or in everyday life can promote the production of energy, thereby making it possible to improve athletic capabilityor to ameliorate fatigue.
As an AMPK activator, endurance improving agent, fatigue-preventing or ameliorating agent, catechins can hence become an ingredient for human or animal foods or drug.

The endurance improving agent or the like according to the present invention can be administered to humans and animals, and in addition, foods and beverages, drug, pet foods and the like containing the endurance improving agent or the like of the present invention can be ingested. Applicable foods include foods and beverages intended to act on physiological functions, for example, to improve endurance or to prevent or ameliorate fatigue, invalid diets, and specific health foods. When employed as drug, it can be formulated, for example, into oral solid preparations such as tablets and granules and oral liquidpreparations such as internal liquid medicines and syrups.
To prepare an oral solidpreparation, an excipient and, if necessary, a binder, disintegrator, lubricant, colorant, taste corrigent, aroma corrigent and/or the like are added to catechins, and the resulting mixture is prepared into tablets, coated tablets, a granule, a ponder, capsules or the like by a method known per se in the art. To prepare an oral liquid preparation, on the other hand, a taste corrigent, buffer, stabilizer, aroma corrigent and/or the like are added, and the resulting mixture is prepared into an internal liquid preparation, a syrup, an elixir or the like by a method known *per se* in the art.

The content of catechins in each of the above-described preparations differs depending on the manner of its used. In the case of a beverage or food, a pet food or the like, the content can generally be set, preferably at from 0.01 to 5 wt%, more preferably from 0.05 to 5 wt%, still more preferably from 0.1 to 1 wt%. In the case of drug other than those described above, for example, an oral solid preparation such as tablets, granules or capsules or an oral liquid preparation such as an internal liquidmedicine or syrup, the content can generally be set, preferably at from 0.01 to 95 wt%, more preferably from 5 to 95 wt%, still more preferably from 10 to 95 wt%.

The daily dose (effective ingestion) of the endurance improving agent or the like according to the present invention can be set preferably at from 100 to 3,000 mg/60 kg-body weight, more preferably at from 250 to 2,000 mg/60 kg-body weight, still more preferably from 250 to 1,000 mg/60 kg-body weight.

### Examples

### Test 1 (endurance improving effect and antifatigue effect of catechins for rats)

As catechins, "POLYPHENON 70S" widely available on the market was obtained from Tokyo Food Techno Co., Ltd., and was used in the test.

After each rat (SD strain, male, 6 weeks old) had been allowed to acclimatize himself to treadmill running for 2 weeks, its maximal running time was measured. The measurement was started after the rat had been allowed to rest in a treadmill and to acclimatize itself to the environment. The belt speedwas set firstly at 12 m/min, and the measurement was conducted at speeds of 12, 15 and 18 m/min, each for 10 minutes, at a speed of 21 m/min for 30 minutes, at a speed of 22.5 m/min for 1 hour, and thereafter, at a speed of 24 m/min. A time point at which the rat became no longer able to run was considered to be its maximal running time, and that time was recorded. To avoid any intergroup difference in maximal running time, the rats were divided into two groups, each consisting of 10 rats. With feeds prepared in accordance with the compositions shown in Table 1, those rats were reared respectively. After reared for 2 weeks, the maximal running time of each group was measured. The maximal running time of the rats at that time are shown in Table 2.

**[Table 1] Feed Composition (wt%)**

| | Test feed | Control feed |
|---|---|---|
| Casein | 20 | 20 |
| DL-methionine | 0.2 | 0.2 |
| Fat | 10 | 10 |
| "POLYPHENONE 70S" | 0.5 | 0 |
| Minerals | 4 | 4 |
| Vitamins | 2.2 | 2.2 |
| Cellulose powder | 8.1 | 8.1 |
| Potato starch | 55 | 55.5 |
| Total | 100 | 100 |

**[Table 2] Maximal running time of rats before and after reared for 2 weeks**

| | Before reared | | After reared for 2 weeks | |
|---|---|---|---|---|
| | Maximal running time (min) | SSD** | Maximal running time (min) | SSD** |
| Control feed | 109.4±15.9 | | 120.7±17.6 | |
| Test feed | 110.1±14.9 | N.S.* | 149.3±26.5 | P<0.05* |

| | | | | |
|---|---|---|---|---|
| * Statistical significance of difference between the test feed and the control feed | | | | |
| ** SSD: Statistical significance of difference | | | | |

It is appreciated from the results of Table 2 that compared with the rats which ingested the control feed, the rats which ingested the feed containing catechins were significantly long in the maximal running time after being reared for 2 weeks and therefore, that endurance improving effect and fatigue suppressing effect were observed on the feed containing catechins.

### Test 2 (endurance improving effect and antifatigue effect of catechins for mice)

As catechins, "POLYPHENON 70S" widely available on the market was obtained from Tokyo Food Techno Co., Ltd., and was used in the test.

After each mouse (BALB/c strain, male, 6 weeks old) had been provisionally reared for 1 week, its maximal swimming time was measured twice. The mouse was caused to swim at a flow rate of 7 L/min. A time point at which the mouse became no longer able to swim was considered to be his maximal swimming time, and that time was recorded. To avoid any intergroup difference in maximal swimming time, the mice were divided into two groups, each consisting of 10 mice. With the feeds prepared in accordance with the compositions shown in Table 1, those mice were reared respectively. While rearing them for 5 weeks, the maximal swimming time of each group was measured once a week. The maximal swimming time of the mice at that time is shown in Table 3.

**[Table 3] Maximal swimming time of mice before and after reared for 4 weeks**

| | Before reared | | After reared for 4 weeks | |
|---|---|---|---|---|
| | Maximal swimming time (min) | SSD** | Maximal swimming time (min) | SSD** |
| Control feed | 26.5±3.73 | | 31.0±6.35 | |
| Test feed | 26.8±3.89 | N.S.* | 40.5±10.8 | P<0.05* |

| | | | | |
|---|---|---|---|---|
| * Statistical significance of difference between the test feed and the control feed | | | | |
| **SSD: Statistical significance of difference | | | | |

It is appreciated from the results of Table 3 that compared with the mice which ingested the control feed, the mice which ingested the feed with catechins added therein were significantly long in the maximal swimming time after reared for 4 weeks and therefore, that endurance improving effect and fatigue suppressing effect were observed on the feed with catechins added therein.

### Test 3 (AMPK activating effect of catechins)

Using a mouse hepatocyte line (Hepa 1-6), activation of AMPK was assessed based on degrees of phosphorylation of AMPK and ACC (acetyl-CoA carboxylase) as indices.
The mouse hepatocyte line (Hepa 1-6) was seeded in a 25-cm² flask, and cultured in DMEM (+10% FBS, +antimicrobial agent) at 37°C for 1 to 2 days. When the culture has become subconfluent, the medium was removed. After washed with PBS(-), DMEM (-FBS) was added as a replacement medium, followed by further culture for 1 day. Subsequent to removal of the medium, DMEM (-FBS) with catechins and a tea extract added at predetermined concentrations therein was added, followed by culture for 60 minutes. Subsequent to removal of the medium and washing with PBS (-), a lytic solution [10 mM Tris (pH 7.4), 50 mM NaCl, 30 mM sodium pyrophosphate, 0.5% Triton X-100, protease inhibitor cocktail ("SIGMA P2714"), phosphatase inhibitor cocktail-1 ("SIGMA P2850"), phosphatase inhibitor cocktail 1-2 ("SIGMA P5726"); 200 µL] was added, and a lysate was collected by a cell scraper. The thus-collected lysate was caused to pass three times through a syringe with 23G needle so that the lysate was homogenized. The homogenized lysate was then allowed stand on ice for 30 minutes. The lysate was centrifuged at 15,000 rpm and at 4°C for 15 minutes, and proteins in supernatant were provided for use in the following experiment.

After the concentration of the proteins in supernatant was measured, it was adjusted such that the protein concentration became identical among the samples. A 1/4 volume of SDS buffer (250 mM Tris, 12.5% SDS, 20% glycerol) was added, followed by further addition of 2-mercaptoethanol and bromophenol blue. The resulting mixture was subjected to thermal denaturation at 95°C, and was then quenched at 4°C to provide a sample for electrophoresis.

A predetermined amount (about 20 to 40 µg) of the sample for electrophoresis was subjected to SDS-PAGE (4 or 12% gel). After transferred onto a membrane, phospho-AMPK α (Thr72), phospho-AMPK β (Ser108) and phospho-ACC (Ser79) were detected using an anti-phospho-AMPK α antibody (product of Cell Signaling Technology, Inc.), an anti-phospho-AMPK β antibody (product of Cell Signaling Technology, Inc.) or an anti-phospho-ACC antibody (product of Cell Signaling Technology, Inc.) as a primary antibody, an anti-rabbit-HRP antibody (product of Amersham Biosciences, Inc.) as a secondary antibody, and a phototope-HRP Western detection system (product of Cell Signaling Technology, Inc.) as a detection reagent . The intensities of detected bands were converted into numerical values (pixels) by an image analysis ("EDAS 290 Image Analysis System", Kodak Company). Supposing that the corresponding band intensities of the control (catechins non-added group) were 100, the numerical values (pixels) were indicated as relative values to the values of the control. It is to be noted that as catechin samples, products of SIGMA Corporation or Wako Pure Chemical Industries, Ltd. were used. As the tea extract (tea catechins), "TEAFURAN" (product of ITO EN, LTD.) as used.

**[Table 4]**

| | Concentration (µM) | Phospho-AM PK α |
|---|---|---|
| Control | 0 | 100 |
| Catechin | 150 | 78 |
| Epicatechin | 150 | 246 |
| Gallocatechin | 150 | 451 |
| Epigallocatechin | 150 | 435 |
| Catechingallate | 150 | 369 |
| Epicatechingallate | 150 | 256 |
| Gallocatechingallate | 150 | 1124 |
| Epigallocatechingallate | 150 | 1085 |

**[Table 5]**

| | Cone. (µM) | Phospho-AMPK α | Phospho-AMPK β | Phospho-ACC |
|---|---|---|---|---|
| Control | 0 | 100 | 100 | 100 |
| Epigallocatechingallate | 100 | 198 | 120 | 220 |
| Epigallocatechingallate | 150 | 261 | 149 | 304 |

**[Table 6]**

| | Conc. (%) | Phospho-AMPK α | Phospho-AMPK β | Phospho-ACC |
|---|---|---|---|---|
| Control | 0 | 100 | 100 | 100 |
| Tea extract | 0.01 | 536 | 124 | 268 |

From the results of Tables 4 to 6, it has been found that catechins have excellent AMPK-activating effect, especially gallocatechingallate, epigallocatechingallate, gallocatechin and epigallocatechinhave excellent AMPK-activatingeffect. It has also become evident that a tea extract containing catechins also exhibits superb AMPK-activating effect.

## Claims

**1.** An endurance improving agent comprising catechins as an active ingredient.

**2.** An antifatigue agent comprising catechins as an active ingredient.

**3.** Use of catechins for the production of an endurance improving agent.

**4.** Use of catechins for the production of an antifatigue agent.

**5.** A method for improving endurance, which comprises administering an effective dose of catechins.

**6.** A method for ameliorating fatigue, which comprises administering an effective dose of catechins.

**7.** An AMPK activator comprising catechins as an active ingredient.

**8.** Use of catechins for the production of an AMPK activator.

**9.** A method of activating AMPK, which comprises administering an effective dose of catechins.
